Europäisches Patentamt

European Patent Office

Office européen des brevets

(19)

(11) Veröffentlichungsnummer: **0 185 279**
**B1**

(12) **EUROPÄISCHE PATENTSCHRIFT**

(45) Veröffentlichungstag der Patentschrift: **06.06.90**

(51) Int. Cl.⁵: **C 07 D 251/10**

(21) Anmeldenummer: **85115595.2**

(22) Anmeldetag: **07.12.85**

(54) **Verfahren zur Herstellung von 1,5-Diacetyl-2,4-dioxohexa-hydro-1,3,5-triazin.**

(30) Priorität: **17.12.84 DD 270940**

(43) Veröffentlichungstag der Anmeldung:
**25.06.86 Patentblatt 86/26**

(45) Bekanntmachung des Hinweises auf die
Patenterteilung:
**06.06.90 Patentblatt 90/23**

(84) Benannte Vertragsstaaten:
**AT DE FR GB IT NL SE**

(56) Entgegenhaltungen:
**DD-A- 229 693**
**DD-A- 229 695**
**DD-A- 229 696**
**DE-A-2 118 281**
**DE-A-2 807 218**

(73) Patentinhaber: **VEB Leuna-Werke "Walter Ulbricht"
DDR-4220 Leuna 3 (DD)**

(72) Erfinder: **Schmidt, Harald, Dr.
Dürerstrasse 15
DDR-4200 Merseburg (DD)**
Erfinder: **Voigt, Dietrich, Dr.
516/2
DDR-4090 Halle-Neustadt (DD)**
Erfinder: **Kiessling, Wolf, Dr.
Otto-Adam-Strasse 11
DDR-7022 Leipzig (DD)**
Erfinder: **Haack, Horst, Dr.
M.E.-Platz 1
DDR-4200 Merseburg (DD)**
Erfinder: **Nitzsche, Reinhard, Dr.
Leninplatz 8
DDR-4220 Leuna (DD)**
Erfinder: **Baumann, Wolfgang
Nr. 6
DDR-4200 Leuna-Göhlitzsch (DD)**
Erfinder: **Liebscher, Gert, Dipl.-Chem.
224/91
DDR-4090 Halle-Neustadt (DD)**
Erfinder: **Schmidtke, Peter
233/8**

Courier Press, Leamington Spa, England.

EP 0 185 279 B1

# EP 0 185 279 B1

(72) DDR-4090 Halle-Neustadt (DD)
Erfinder: **Enge, Günter, Dipl.-Chem.**
**Akazienweg 19**
**DDR-4202 Merseburg (DD)**
Erfinder: **Esser, Gerhard, Dr.**
**Lilienweg 2**
**DDR-4220 Leuna (DD)**
Erfinder: **Winter, Harald, Dr.**
**Windmühlenstrasse 10**
**DDR-4220 Leuna (DD)**

(74) Vertreter: **Puchberger, Rolf, Dipl. Ing. et al**
**Patentanwälte, Dipl. Ing. Georg Puchberger Dipl.**
**Ing. Rolf Puchberger Dipl. Ing. Peter Puchberger**
**Singerstrasse 13 Postfach 55**
**A-1010 Wien (AT)**

# EP 0 185 279 B1

**Beschreibung**

Die Erfindung betrifft ein Verfahren zur Herstellung von 1,5-Diacetyl-2,4-dioxo-hexahydro-1,3,5-triazin (DADHT) durch Acetylierung von 2,4-Dioxo-hexahydro-1,3,5-triazin (DHT). Das DADHT wird vor allem Waschmitteln als Bleichmittelaktivator zugesetzt.

Die Acetylierung von NH-Verbindungen, z.B. von Aminen und Säureamiden, mit Essigsäure, Acetanhydrid und/oder Keten ist eine bereits lange bekannte und auch für unterschiedliche technische Zwecke genutzte Reaktion (vgl. P. F. G. Praill, Acylierungsreaktionen, Pergamon Press, New York 1963). Verläuft die Monoacetylierung von Aminen selbst mit Essigsäure mit befriedigenden Resultaten, so bedarf es für die erschöpfende Acetylierung von NH-Verbindungen, insbesondere bei Di- und Polyaminen, besonderer Maßnahmen, um einen hohen Umsatz zu erreichen.

Es ist bekannt, zur vollständigen Acetylierung von Diaminen, wie beispielsweise Äthylendiamin und Methylendiamin, als besonders reaktionsfähiges Acetylierungsmittel Keten einzusetzen, wobei aber in Gegenwart von Katalysatoren (US—PS 3 223 732, US—PS 3 228 983, DE—OS 1 910 300) und in lösung (DE—AS 1 200 798, DE—AS 2 308 119, DE—AS 2 052 822) oder in der Schmelze (DE—AS 1 668 853) gearbeitet werden muß, ohne daß ein vollständiger Umsatz und eine befriedigende Reaktionsgeschwindigkeit erreicht werden. Darüberhinaus wurden verunreinigte Acetylierungsprodukte erhalten. Die Handhabung des gasförmigen Ketens verlangt wegen seiner Toxizität und der Neigung zur Dimerisierung besondere Vorsichtsmaßnahmen.

Um die Nachteile einer Acetylierung mit Keten zu vermeiden, wurde mehrfach auch Acetanhydrid für eine erschöpfende Acetylierung herangezogen. So its es bekannt, Diamine erschöpfend mit Acetanhydrid zu acetylieren bei Anwendung eines Acetanhydridüberschusses bis zu 30 Mol/Mol Amin (DE—AS 1 149 349, DE—AS 2 118 281), wobei ferner ein Lösungsmittel (DE—OS 2 636 865) und Temperaturen im Bereich von 120 bis 190°C (393 bis 463 K), überwiegend im Bereich von 150 bis 190°C (423 bis 463 K), (DE—AS 2 118 281, DE—AS 2 906 606, DE—OS 3 042 148) anzuwenden sind und mit einem Teilumsatz (DE—OS 2 846 174, DE—OS 2 906 606) gearbeitet wird.

Durch die Ausführung der Acetylierung bei hohen Temperaturen, auch mit gelösten und/oder geschmolzenen Substanzen, werden dunkelgefärbte und unreine Reaktionsprodukte erhalten, die nachträglich gereinigt werden müssen. So beispielsweise durch adsorptive Behandlung von Lösungen der Acetylierungsprodukte (DE—AS 2 826 174), Rekristallisation (DE—AS 2 636 865, DE—OS 3 042 148) und andere aufwendige Operationen, wie beispielsweise das Suspendieren in einem Lösungsmittel (DE—OS 3 127 435).

Weiterhin ist es bekannt, NH-Verbindungen, auch DHT, erschöpfend zu acetylieren durch Kochen mit einem erheblichen Überschuß von Acetanhydrid (Rec. Trav. Chim. 30 (1911), 183; Coll. Czech. Chem. Commun 27 (1962), 1562—7). Die dabei notwendigen langen Reaktionszeiten führen zu verfärbten Produkten, die einer nachträglichen aufwendigen Reinigung bedürfen.

Zur Erhöhung des Acetylierungsgrades bzw. des Umsatzes der zu acetylierenden Verbindung wurde auch vorgeschlagen, die bei der Arbeitsweise mit Acetanhydrid entstehende Essigsäure aus dem Reaktionsgemisch durch Abdestillieren ständig zu entfernen (EP 79 671). Das Erreichen diese Zieles ist bei allen bekannten technischen Lösungen mit wesentlichen Nachteilen verbunden. So führen die unter Verwendung der bekannten Katalysatoren angewandten Verfahren der benötigten hohen Temperaturen für die destillative Entfernung der gebildeten Essigsäure zu starck dunkelgefärbten Produkte, die noch nachträglich gereinigt werden müssen. Von Nachteil ist auch der hohe Acetanhydridüberschuß, um die Reaktionsprodukte vollständig in Lösung zu halten (DE—OS 2 133 458) sowie die Entfernung erheblicher Mengen an Acetanhydrid zusammen mit der Essigsäure (EP 79 671, DE—OS 2 118 281) und die Acetylierung in einer Rührkesselkaskade mit hohem technischen Aufwand (DE—OS 2 118 281) durchzuführen.

Die bekannten Verfahren zur erschöpfenden Acetylierung von NH-Verbindungen unter Entfernung des Reaktionsproduktes Essigsäure können also wegen der Verfärbung des Reaktionsproduktes, des hohen Acetanhydridbedarfs und der aufwendigen Reaktionsführung nicht befriedigen.

Ziel der Erfindung ist es, auf ökonomische und technisch einfache Weise DADHT herzustellen, das den Reinheitsanforderungen, die an einen Waschmittelzusatz zu stellen sind, ohne Einschränkungen entspricht.

Es besteht somit die Aufgabe, für die Acetylierung von DHT solche Bedingungen anzugeben, daß mit einem geringen Überschuß des Acetylierungsmittels Acetanhydrid ein praktisch quantitativer Umsatz des DHT in kurzer Zeit erreicht wird und das Zielprodukt DADHT ohne aufwendige Reinigung sofort als Bleichmittelaktivator Waschmittelkompositionen zugesetzt werden kann, das DADHT darf also nicht verfärbt und muß ökonomisch herstellbar sein.

Diese Aufgabe wird durch ein Verfahren zur Herstellung von DADHT durch Acetylierung von DHT mit Acetanhydrid gelöst, indem erfindungsgemäß das in Acetanhydrid und/oder Mutterlauge der DADHT-Herstellung suspendierte DHT zusammen mit Alkalihydroxid, insbesondere von Natriumhydroxid, in einem Molverhältnis DHT zu Alkalihydroxid von 5 bis 40, vorzugsweise 10 bis 15, unter gleichzeitiger fraktionierter Destillation der sich bei der Acetylierung bildenden Essigsäure auf eine Temperatur von 90 bis 150°C (363 bis 423 K) erhitzt wird, wobei die Kopftemperatur der Destillationskolonne 97 bis 118°C (370 bis 391 K) bei 65 bis 101 kPa bei einem Rücklaufverhältnis von 2 bis 8, vorzugsweise 3 bis 5, beträgt und die Acetylierungsreaktion solange betrieben wird, bis 70 bis 100%, vorzugsweise 80 bis 95%, der theoretisch

3

zu erwartenden Essigsäuremenge abdestilliert sind und die Konzentration der Essigsäure im Reaktionsgemisch 5 bis 20 Ma.-%, vorzugsweise 5 bis 12 Ma.-%, nicht überschreitet.

Die fraktionierte Destillation der Essigsäure nach dem erfindungsgemäßen Verfahren erfolgt über eine Kolonne mit einem hohen Durchsatz mit 10 bis 20, vorzugsweise 12 bis 15, theoretischen Boden, so daß die bei der Acetylierung entstehende Essigsäure in kurzer Zeit und hoher Reinheit abdestilliert werden kann.

Nach dem erfindungsgemäßen Verfahren werden an das DHT keine hohen Qualitätsanforderungen gestellt, es kann Verunreinigungen an Cyanursäure, Methylenbisharnstoff, Harnstoff, u.a. enthalten, die aber in der Summe 15 Ma.-% nicht übersteigen sollen. Die Jodfarbzahl des DHT soll kleiner als 3, vorzugsweise kleiner als 2, sein. (Farbzahl wird bestimmt von einer 1,6 Ma.-% DHT enthaltenden Lösung in 2%iger Kalilauge). Das zu acetylierende DHT ist unter den Bedingungen des erfindungsgemäßen Verfahrens nur zu einem geringen Teil gelöst. Auch das DADHT als Reaktionsprodukt der Acetylierung ist unter den Bedingungen des erfindungsgemäßen Verfahrens zum überwiegenden Teil als Kristalle abgeschieden. Es ist deshalb vorteilhaft, das Reaktionsgemisch während der Acetylierungsreaktion auf bekannte Weise zu durchmischen.

Für die Dosierung des Alkalihydroxids zu dem Acetylierungsgemisch kann dieses sowohl in fester Form als auch in Lösung eingestezt werden. Besonders vorteilhaft nach dem erfindungsgemäßen Verfahren und überdies technisch besonders einfach ist es, die Alkalihydroxide als hochkonzentrierte, wäßrige Lösung zu verwenden, beispielsweise als Natronlauge mit 50 Ma.-% NaOH.

Für die Bereitung der Suspension von DHT kann sowohl Acetanhydrid als auch Mutterlauge, die bei der Isolierung der DADHT anfällt, sowie Gemische beider verwendet werden, wobei geringe Essigsäuregehalte sowohl des Acetanhydrids als auch der Mutterlauge den Acetylierungsablauf nicht beeinträchtigen, sondern nur den Destillatanfall vergrößern. Der Feststoffanteil in der Suspension sollte nicht mehr als 50 Ma.-% erreichen, um das Gemisch ausreichend rühr- und förderbar zu halten.

Wegen der für den Erhalt von praktisch farblosem DADHT genauen Einhaltung des Temperaturregimes bei der Acetylierung und ihres exothermen Verlaufs ist es zweckmäßig, den Acetylierungsreaktor sowohl mit Einrichtungen zum Heizen als auch zum Kühlen auszustatten.

Nach beendeter Acetylierung wird das Reaktionsgemisch auf ca. 25°C (298 K) zur nahezu vollständigen Kristallisation des DADHT abgekühlt, anschließend Kristallmasse und Mutterlauge durch bekannte Maßnahmen, wie beispielsweise Zentrifugieren, voneinander getrennt, und der Filterkuchen ein- bis dreimal mit Essigsäure gewaschen. Die Mutterlauge wird auf die bereits beschriebene Art für die Bereitung der DHT-Suspension verwendet. Die Waschfiltrate werden durch Destillation aufgearbeitet, wobei aus dem Sumpf weiteres DADHT isoliert werden kann.

Das DADHT wird auf bekannte Art getrocknet und durch die Bestimmung von Acetylierungsgrad, Schmelzpunkt, Farbzahl und Natriumgehalt charakterisiert (Farbzahl wird bestimmt in einer 1,6 Ma.-% DADHT enthaltenden Lösung in 2%iger Natronlauge).

Ausführungsbeispiele

1. In einem heiz- und kühlbaren Rührbehälter mit einem Inhalt von 30 l und einer aufgesetzten Kolonne (Durchmesser 80 mm, Länge 2000 mm, Füllkörper Schaumkeramik, Korngröße 5 mm, ca. 15 theoret. Böden) mit Dephlegmator und Destillatteiler sowie Einrichtungen zur Temperaturkontrolle im Rührbehälter und der Destillationseinrichtung wurden 4,0 kg DHT (DHT-Gehalt 94,0 Ma.-%, Cyanursäuregehalt 5,2 Ma.-%) in 20 kg Acetanhydrid (Gehalt 98,0 Ma.-%) suspendiert und zu diesem Gemisch 0,13 kg 50%ige Natronlauge zugesetzt. Das Acetylierungsgemisch wird zunächst bei 100 kPa auf 135°C (408 K) unter Rückfluß aufgeheizt und bei dieser Temperatur und einem Rücklaufverhältnis vo 3,5 sowie der Kopftemperatur von 116°C (389 K) die Essigsäure abdestilliert. Über einen Destillationszeitraum von 5 Stunden fallen 3,6 kg Essigsäure an, wobei sich gleichzeitig die milchige Suspension von DHT in Acetanhydrid in einen Kristallbrei von DADHT verwandelt.

Danach wird die Acetylierungsreaktion abgebrochen, das Reaktionsgemisch auf 25°C (298 K) gekühlt und das DADHT durch Absaugen in einer Filternutsche von der Mutterlauge getrennt. Das DADHT wurde zweimal mit Essigsäure gewaschen und im Vakuum bei 80°C (353 K) getrocknet.

Es wurden 6,02 kg trockenes DADHT, entsprechend 88,8 Mol-%, bezogen auf DHT, mit den folgenden Kennzahlen isoliert:

| | |
|---|---|
| Acetylierungsgrad | 95,8% |
| Jodfarbzahl | 0,6 |
| Schmelzpunkt | 215°C (488 K) |
| Gehalt Cyanursäure | 0,9 Ma.-% |
| Natrium | 0,02 Ma.-% |

2—6 Beispiel

Die Reaktionsbedingungen, Mengenverhältnisse und Ergebnisse der Ausführungsbeispiele 2 bis 6 enthält die folgende Tabelle. Die Arbeitsweise bei der Acetylierung und die verwendete Apparatur entsprechen dem Ausführungsbeispiel 1.

Bei den Ausführungsbeispielen 2 bis 5 wurde jeweils die bei der DADHT-Isolierung anfallende Mutterlauge des vorhergehenden Ausführungsbeispieles neben Acetanhydrid zum Suspendieren des DHT verwendet.

TABELLE

| Ausführungsbeispiele 2 bis 6<br>Ausführungsbeispiel | 2 | 3 | 4 | 5 | 6 |
|---|---|---|---|---|---|
| **Reaktionsbedingungen** | | | | | |
| Temp. Acetylierungsgemisch °C | 138 | 132 | 125 | 125 | 132 |
| K | 411 | 405 | 398 | 398 | 405 |
| Kolonnenkopf °C | 116 | 108 | 102 | 102 | 115 |
| K | 389 | 381 | 375 | 375 | 388 |
| Druck kPa | 102 | 90 | 84 | 82 | 102 |
| Rücklaufverhältnis | 3 | 3 | 4 | 2,5 | 3 |
| Trennstufenzahl | 15 | 15 | 15 | 15 | 12 |
| Reaktionszeit h | 4 | 6 | 8 | 5 | 6 |
| Destillatanfall kg | 4,6 | 4,8 | 4,2 | 4,4 | 4,5 |
| **Einsatzmengen** | | | | | |
| DHT kg | 4,0 | 4,0 | 4,0 | 4,0 | 4,0 |
| mit DHT Ma.-% | 94,0 | 94,0 | 94,0 | 94,0 | 91,2 |
| Cyanursäure Ma.-% | 5,2 | 5,2 | 5,2 | 5,2 | 8,0 |
| Acetanhydrid kg (98%ig) | 9,0 | 11,0 | 11,5 | 11,0 | 20,0 |
| Mutterlauge von Beispiel | 1 | 2 | 3 | 4 | — |
| Menge kg | 11,0 | 9,0 | 8,5 | 9,0 | — |
| mit Acetanhydrid Ma.-% | 84,7 | 87,5 | 88,0 | 87,3 | — |
| Essigsäure Ma.-% | 7,6 | 4,4 | 2,5 | 4,1 | — |
| Natronlauge Konz. Ma.-% | 50 | 50 | 50 | 50 | 50 |
| Menge kg | 0,13 | 0,08 | 0,08 | 0,20 | 0,15 |
| **Ergebnis** | | | | | |
| DADHT-Anfall kg | 5,82 | 5,96 | 5,83 | 5,76 | 5,64 |
| DADHT Acetylierungsgrad % | 95,2 | 96,2 | 94,1 | 93,8 | 96,2 |
| Cyanurgehalt Ma.-% | 1,2 | 1,2 | 1,4 | 1,5 | 2,4 |
| Schmelzpunkt °C | 213—215 | 212—214 | 212—214 | 208—214 | 212—215 |
| K | 486—488 | 485—487 | 485—487 | 481—487 | 485—488 |
| Jodfarbzahl | 1,2 | 1,4 | 1,3 | 1,4 | 0,8 |
| Natriumgehalt Ma.-% | 0,02 | 0,02 | 0,02 | 0,04 | 0,02 |
| DADHT-Ausbeute Mol-%<br>(bez. auf DHT) | 85,9 | 88,3 | 84,5 | 83,2 | 86,0 |

**Patentansprüche**

1. Verfahren zur Herstellung von 1,5-Diacetyl-2,4-dioxo-hexahydro-1,3,5-triazin (DADHT) durch Acetylierung von 2,4-Dioxo-hexahydro-1,3,5-triazin (DHT) mit Acetanhydrid, dadurch gekennzeichnet, daß das in Acetanhydrid und/oder Mutterlauge der DADHT-Herstellung suspendierte DHT zusammen mit Alkalihydroxid, in einem Molverhältnis von DHT zu Alkalihydroxid von 5 bis 40 unter gleichzeitiger fraktionierter Destillation der sich bei der Acetylierung bildenden Essigsäure auf eine Temperatur von 90 bis 150°C (363 bis 423 K) erhitzt wird, wobei die Kopftemperatur der Destillationskolonne 97 bis 118°C (370 bis 391 K) bei 65 bis 101 kPa bei einem Rücklaufverhältnis von 2 bis 8 beträgt und die Acetylierungsreaktion soweit betrieben wird, bis 70 bis 100% der theoretisch zu erwartenden Essigsäuremenge abdestilliert sind und die Konzentration der Essigsäure im Reaktionsgemisch 5 bis 20 Ma.-% nicht überschreitet.

2. Verfahren nach Anspruch 1, dadurch gekennzeichnet, daß das Molverhältnis von DHT zu Alkalihydroxid 10 bis 15 beträgt.

3. Verfahren nach den Ansprüchen 1 und 2, dadurch gekennzeichnet, daß als Alkalihydroxid Natriumhydroxid eingesetzt wird.

4. Verfahren nach Anspruch 3, dadurch gekennzeichnet, daß das Natriumhydroxid in Form einer hochkonzentrierten wäßrigen Lösung eingesetzt wird.

5. Verfahren nach den Ansprüchen 1 bis 4, dadurch gekennzeichnet, daß das Rücklaufverhältnis 3 bis 5 beträgt.

6. Verfahren nach den Ansprüchen 1 bis 4, dadurch gekennzeichnet, daß 80 bis 95% der theoretisch zu erwartenden Essigsäure abdestilliert werden.

7. Verfahren nach den Ansprüchen 1 bis 4, dadurch gekennzeichnet, daß die Konzentration der Essigsäure im Reaktionsgemisch 5 bis 12 Ma.-% nicht überschreitet.

**Revendications**

1. Procédé de fabrication de la 1,5-diacétyl-2,4-dioxo-hexahydro-1,3,5-triazine (DADHT) par acétylation de la 2,4-dioxo-hexahydro-1,3,5-triazine (DHT) à l'acétanhydrure, caractérisé en ce que la DADHT qui est placée en suspension dans l'acétanhydrure et/ou dans les eaux mères de la fabrication de la DHT est chauffée, avec un hydroxyde alcalin, dans un rapport molaire allant de 5 à 40, entre la DHT et l'hydroxyde alcalin, avec distillation fractionnée simultanée de l'acide acétique se formant lors de l'acétylation, à une température allant de 90 à 150°C (363 à 423°K), la température de tête de la colonne de distillation allant de 97 à 118°C (370 à 391°K) pour une pression allant de 65 à 101 kPa, dans le cas d'un taux de recyclage allant de 2 à 8, et la réaction d'acétylation étant poursuivie jusqu'à ce que 70 à 100% e de la quantité d'acide acétique que l'on peut théroriquement obtenir soit distillé et que la concentration de l'acide acétique dans le mélange réactif ne dépasse pas 5 à 20 MA%.

2. Procédé selon la revendication 1, caractérisé en ce que le rapport molaire entre la DHT et l'hydroxyde alcalin est de 10 à 15.

3. Procédé selon les revendications 1 et 2, caractérisé en ce que l'hydroxyde alcalin utilisé est l'hydroxyde de sodium.

4. Procédé selon la revendication 3, caractérisé en ce que l'hydroxyde de sodium est mis en oeuvre sous la forme d'une solution aqueuse de concentration élevée.

5. Procédé selon les revendications 1 à 4, caractérisé en ce que le taux de recyclage est de 3 à 5.

6. Procédé selon les revendications 1 à 4, caractérisé en ce que 80 à 95% de l'acide acétique que l'on peut obtenir théoriquement est distillé.

7. Procédé selon les revendications 1 à 4, caractérisé en ce que la concentration de l'acide acétique dans le mélange réactif ne dépasse pas 5 à 12 Ma %.

**Claims**

1. Process for the preparation fo 1,5-diacetyl-2,4-dioxo-hexahydro-1,3,5-triazine (DADHT) by acetylation of 2,4-dioxohexahydro-1,3,5-triazine (DHT), with acetic anhydride, characterized in that the DHT, suspended in acetic anhydride and/or mother liquor from the preparation of DADHT, is heated together with alkali metal hydroxide to a temperature of 90 to 150°C (363 to 423 K) in a molar ratio of DHT to alkali metal hydroxide of 5 to 40 with simultaneous fractional distillation of the acetic acid forming during the acetylation, the head temperature of the distillation column being 97 to 118°C (370 to 391 K) at 65 to 101 kPa at a reflux ratio of 2 to 8 and the acetylation reaction being carried on until 70 to 100% of the amount of acetic acid to be expected theoretically has distilled off and the concentration of the acetic acid in the reaction mixture does not exceed 5 to 20 mass-%.

2. Process according to Claim 1, characterized in that the molar ratio of DHT to alkali metal hydroxide is 10 to 15.

3. Process according to Claims 1 and 2, characterized in that sodium hydroxide is employed as the alkali metal hydroxide.

4. Process according to Claim 3, characterized in that the sodium hydroxide is employed in the form of a highly concentrated aqueous solution.

5. Process according to Claims 1 to 4, characterized in that the reflux ratio is 3 to 5.

6. Process according to Claims 1 to 4, characterized in that 80 to 95% of the acetic acid to be expected theoretically is distilled off.

7. Process according to Claims 1 to 4, characterized in that the concentration of the acetic acid in the reaction mixture does not exceed 5 to 12 mass-%.